# EUROPEAN PATENT SPECIFICATION

(11) **EP 3 394 510 B1**
(45) Date of publication and mention of the grant of the patent: **14.09.2022**
(21) Application number: 16878602.8
(22) Date of filing: 19.12.2016
(51) Int. Cl.: F22B 27/00, F22B 1/28, B81B 1/00, B81B 7/00, F04B 19/00, B01L 3/00, A61M 11/04, A61M 15/00, A61M 15/06, F04B 19/06, F04B 19/24

(54) **VAPORIZATION DEVICE**
VERDAMPFUNGSVORRICHTUNG
DISPOSITIF DE VAPORISATION

(30) Priority: 21.12.2015 US 201514976053; 21.12.2015 US 201514976067
(43) Date of publication of application: 31.10.2018
(73) Proprietor: Funai Electric Co., Ltd., Daito-shi Osaka 574-0013 (JP)
(72) Inventor: BARKLEY, Lucas D., Daito-shi Osaka 574-0013 (JP)
(74) Representative: Isarpatent
(86) International application number: PCT/JP2016/087716
(87) International publication number: WO 2017/110713

(56) References cited:
- EP-A2- 0 309 199
- WO-A1-2016/064684
- JP-A- H09 196 302
- JP-A- 2004 061 010
- JP-A- 2004 061 010
- US-A- 5 599 502
- US-A1- 2003 086 790
- US-A1- 2004 086 816
- US-A1- 2006 005 437
- US-A1- 2014 051 159
- US-A1- 2015 108 109
- US-A1- 2016 007 653
- US-B2- 8 891 949

## Description

### Technical Field

The present invention relates generally to methods and apparatus for metering and vaporizing a fluid. More particularly, this invention relates to fluid vaporization structures that utilize a bubble pump to transport fluid to a vaporization structure.

### Background Art

Improvement is desired in the field of microfluidic structures of the type used to dispense a solution from a storage supply to another device where a secondary function may be performed. An example of one secondary function is vaporization of the solution using a heater such that the contents of the solution can be delivered to complete its function in a gaseous state. Such microfluidic structures have many applications, such as for providing vapor therapy, flavored e-cigarettes, chemical vapor reactions, and the like.

US 2014/051159 A1 describes a chip comprising a bubble pump including a flow channel and a heater layer. Further, vaporizing devices are described for example in U.S.Pat.No.8,891,949 and JP 2004 061010 A.

### Summary of Invention

### Technical Problem

Conventional structures for dispensing fluid from a fluid supply to a vaporization heater structure desire improvement. For example, conventional devices are often unreliable in providing consistent and desired amounts of fluid to the vaporization heater structure. As part of this, clogging of the flow path and causes of incomplete travel of fluid are common, resulting in uncertainty of the amount of fluid that reaches the vaporizing element.

The present invention advantageously provides improved apparatus for metering and vaporizing fluids.

### Solution to Problem

The present invention provides a vaporization device in accordance with claim 1

### Advantageous Effects of Invention

The vaporization device according to the present invention is reliable in providing consistent and desired amounts of fluid to the vaporization heater structure.

### Brief Description of Drawings

Further advantages of the invention are apparent by reference to the detailed description in conjunction with the figures, wherein elements are not to scale so as to more clearly show the details, wherein like reference numbers indicate like elements throughout the several views, and wherein:
FIG. 1 shows a fluid vaporization device according to an example which is not part of the present invention, wherein a vaporizer is located in a plane substantially parallel to a plane defined by a bubble pump;
FIG. 2 shows a fluid vaporization device according to an example which is not part of the present invention, wherein a vaporizer is located in a plane substantially parallel to a plane defined by a bubble pump;
FIG. 3 shows a fluid vaporization device according to an example which is not part of the present invention, wherein a vaporizer is located in a plane substantially parallel to a plane defined by a bubble pump;
FIG. 4 shows an alternate example of fluid vaporization device not part of the invention, wherein a vaporizer is located in a plane substantially perpendicular to a plane defined by a bubble pump;
FIG. 5 shows yet another alternate embodiment of fluid vaporization device according to the invention in which an angle between a plane defined by vaporizers and a plane defined by a bubble pumps is varied;
FIG. 6 shows yet another alternate embodiment of fluid vaporization device according to the invention in which an angle between a plane defined by vaporizers and a plane defined by a bubble pumps is varied;
FIG. 7 shows a further example of a fluid vaporization device not forming part of the present invention, the device having a fluid supply inlet located at an edge of the device;
FIG. 8 shows a still further embodiment of a fluid vaporization device according to the present invention, the device having a fluid supply inlet located at an edge of the device, with an angle between a plane defined by a vaporizer and a plane defined by a bubble pump of the device is varied;
FIG. 9 shows another example of a fluid vaporization device not forming part of the present invention, wherein the bubble pump and the vaporizer are fabricated on the same substrate;
FIG. 10 shows another example of a fluid vaporization device not forming part of the present invention, wherein the bubble pump and the vaporizer are fabricated on the same substrate;
FIG. 11 shows a fluid vaporization device according to an example not forming part of the present invention, wherein a vaporizer is located in a plane substantially parallel to a plane defined by a plurality of bubble pumps;
FIG. 12 shows a fluid vaporization device according to an example not forming part of the present invention, wherein a vaporizer is located in a plane substantially parallel to a plane defined by a plurality of bubble pumps;
FIG. 13 shows an alternate example of fluid vaporization device not forming part of the present invention, wherein a vaporizer is located in a plane substantially perpendicular to a plane defined by the bubble pumps;
FIG. 14 shows yet another alternate embodiment of a fluid vaporization device according to the invention, wherein an angle between a plane defined by vaporizers and a plane defined by a plurality of bubble pumps is varied;
FIG. 15 shows yet another alternate embodiment of a fluid vaporization device according to the invention, wherein an angle between a plane defined by vaporizers and a plane defined by a plurality of bubble pumps is varied;
FIG. 16 shows a further example of a fluid vaporization device not part of the present invention, the device having a fluid supply inlet located at an edge of the device;
FIG. 17 shows a still further embodiment of a fluid vaporization device according to the invention, the device having a fluid supply inlet located at an edge of the device, with an angle between a plane defined by a vaporizer and a plane defined by a plurality of bubble pumps of the device is varied;
FIG. 18 shows another alternate example of a fluid vaporization device not part of the present invention, the device having multiple bubble pumps and multiple fluid supplies;
FIG. 19 shows a further alternate example of a fluid vaporization device not part of the present invention, the device having multiple bubble pumps, with each bubble pump having its own fluid supply;
FIG. 20 shows another example of a fluid vaporization device not part of the present invention, wherein the bubble pumps and the vaporizer are fabricated on the same substrate; and
FIG. 21 shows another example of a fluid vaporization device not part of the present invention, wherein the bubble pumps and the vaporizer are fabricated on the same substrate.

### Description of Embodiments

### (Embodiment 1)

The present invention relates to fluid vaporization structures that utilize one or more bubble pumps to transport fluid from one or more fluid supplies to a discrete fluid vaporization structure.

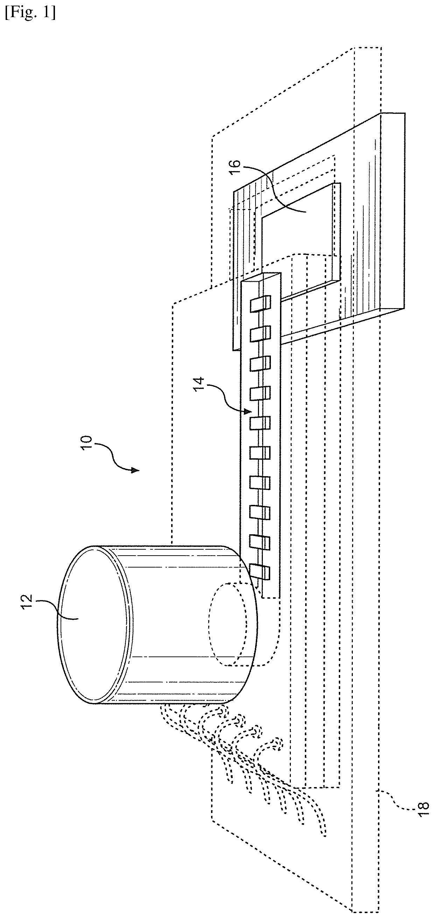

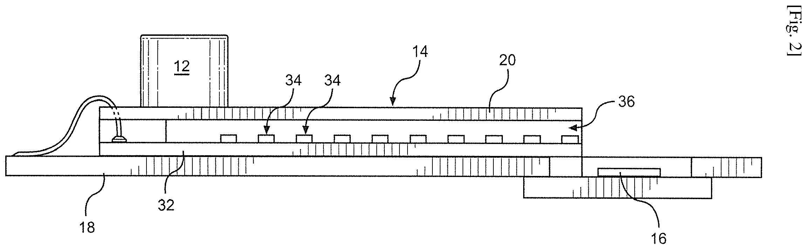

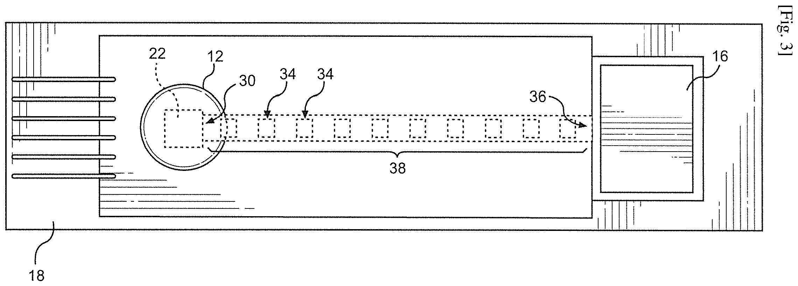

With reference to FIGS. 1-3, there is shown a fluid vaporization device 10 having a fluid supply 12, a bubble pump 14, and a vaporizer 16 (fluid vaporization heater). The device 10 is configured so that the bubble pump 14 desirably transports fluid from the fluid supply 12 directly onto the vaporizer 16.

The device 10 is incorporated onto a printed circuit board 18 to provide a single assembly containing the fluid supply 12, the bubble pump 14, and the vaporizer 16. The bubble pump 14 has a length axis that generally defines a plane, and the vaporizer 16 is provided on a substrate generally defining a plane. As will be noted, in the embodiment of FIGS. 1-3, the plane defined by the bubble pump 14 and the plane defined by the vaporizer 16 are substantially parallel to one another.

The fluid supply 12 is configured as a fluid storage vessel located on a cover substrate 20 of the bubble pump 14. The fluid supply 12 is charged with a desired vaporizable fluid and is generally vented to the atmosphere and contains a desired volume of a fluid, typically a liquid at ambient conditions. As one example, the fluid may be a liquid of a type utilized for vapes or e-cigarettes in a volumetric amount suitable for such usage. A supply inlet 22 is defined between the fluid supply 12 and the cover substrate 20 to provide a fluidic path for desired travel of fluid from the fluid supply12 to the bubble pump 14.

The bubble pump 14 is configured for pumping fluid from the fluid supply 12 to the vaporizer 16. In addition to the cover substrate 20, the bubble pump 14 includes an inlet 30, a base substrate 32, flow sequencing resistive heaters 34, and an outlet 36. During manufacture, a flow feature layer is initially deposited on the base substrate 32. The flow feature layer is then selectively etched to provide the heaters 34 and to define a flow channel 38.

The base substrate 32 may be a semiconductor silicon substrate that is suitable for providing bubble pumps and logic circuits thereon. The cover substrate 20 may be made of silicon or a polymeric material such as polyimide. The resistive heaters 34 and vaporizer 16 may be made of TaAIN, TaAl or other thin film resistor material. The preferred material for the flow feature layer for providing the resistive heaters 34 is TaAIN deposited on the base substrate 32 as by sputtering. The vaporizer 16 may be formed in a similar manner.

Electrical connections and logic circuits are integrated onto the device 10 to control and operate the heaters 34 of the bubble pump 14 and the vaporizer 16, and to otherwise control the transfer of fluid from the fluid supply 12 to the vaporizer 16. For example, voltage pulses may be applied to the heaters 34 in a desired manner to form and transport thermal bubbles of the fluid along the flow channel 38 to deliver fluid as desired to the vaporizer 16 for vaporization of the delivered fluid. Examples of preferred bubble pumps are shown in U.S. Patent No. 8,891,949, issued November 18, 2014, entitled Micro-fluidic pump, and incorporated by reference herein in its entirety.

In basic operation of the bubble pump 14, a voltage pulse is applied to each of the heaters 34 in sequence to generate thermal bubbles in a predetermined manner. For example, every heater 34 can form a bubble from the inlet 30 to the outlet 36 of the channel 38 in sequence to transport fluid as desired from the supply 12 to the vaporization heater 16. Each heater 34 is also desirably permitted to cool down before the next firing sequence in order to prevent overheating and boiling of fluid within the bubble bump 14.

The vaporizer 16 is configured as a microfluidic electrical heating element designed specifically to vaporize the fluid received from the fluid supply 12. The vaporizer 16 is located adjacent and below the outlet 36 of the bubble pump 14. A slot or other flow path is formed through the circuit board 18 for travel of fluid from the outlet 36 of the bubble pump 14 to the vaporizer 16. The vaporizer 16 has a heated fluid contact surface that is open and exposed to the air or other local environment. The heated fluid contact surface heats the received fluid to vaporize the received fluid into the atmosphere or other local environment.

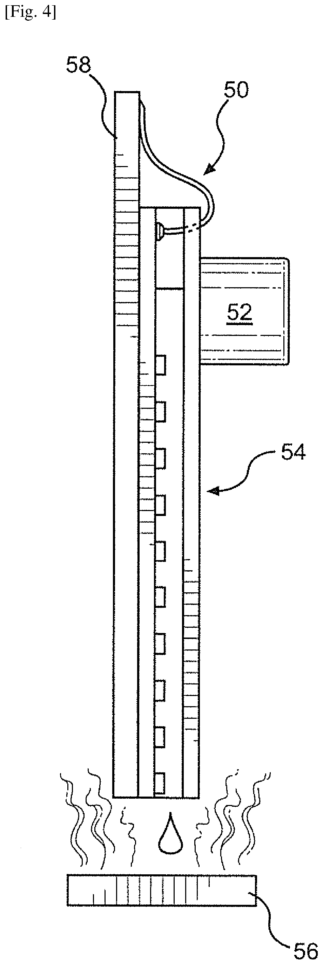

Turning now to FIG. 4, there is shown an alternate embodiment of a fluid vaporization device 50. The device 50 has a fluid supply 52, a bubble pump 54, and a vaporizer 56. The fluid supply 52 and the bubble pump 54 are incorporated onto a printed circuit board 58. The fluid supply 52, the bubble pump 54, and the vaporizer 56 substantially correspond to the fluid supply 12, the bubble pump 14, and the vaporizer 16. However, the vaporizer 56 is spaced from the end of the circuit board 58 so as to be in a plane that is substantially perpendicular to a fluid flow plane defined by the bubble pump 54.

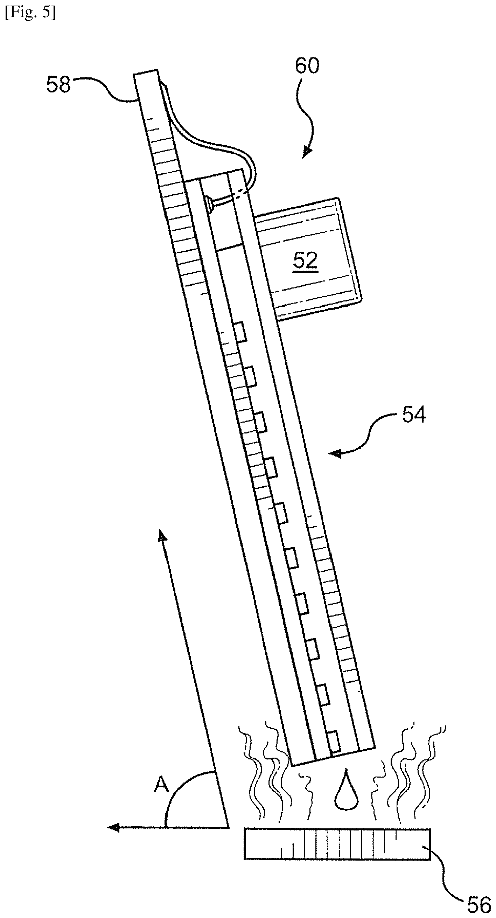

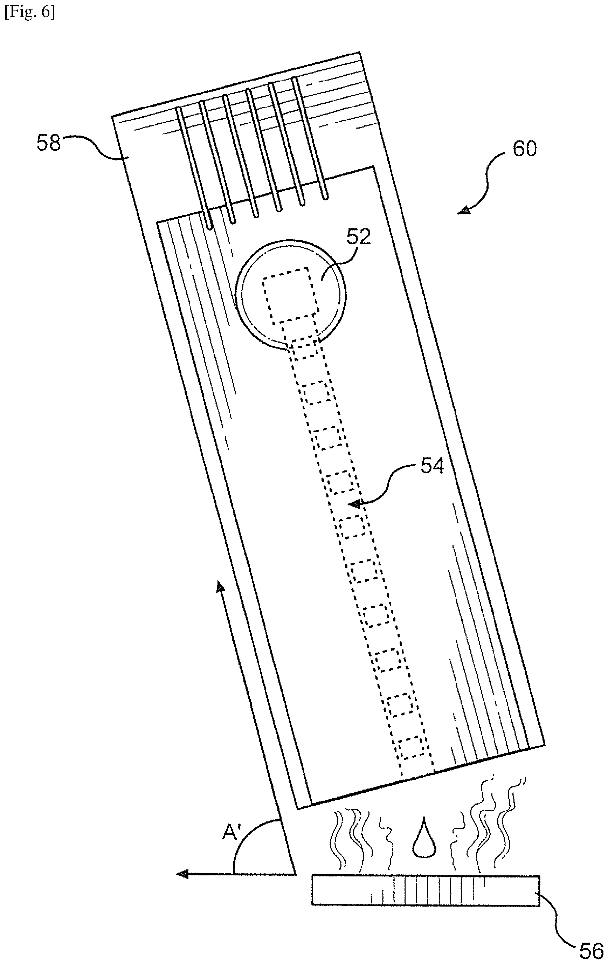

Turning now to FIGS. 5 and 6, there is shown another alternate embodiment of a fluid vaporization device 60. The device 60 substantially corresponds to the device 50, and includes the fluid supply 52, bubble pump 54, and the vaporizer 56, except the circuit board 58 with the bubble pump 54 thereon is oriented at an angle A or an angle A' or both relative to a plane defined by the vaporizer 56. The angles A and A' may each vary from about 0 degrees to about 90 degrees. In this regard, it will be appreciated that the depicted angles are provided to show that the angular orientation between the bubble pump 54 and the vaporizer 56 may be varied in any of the three dimensions.

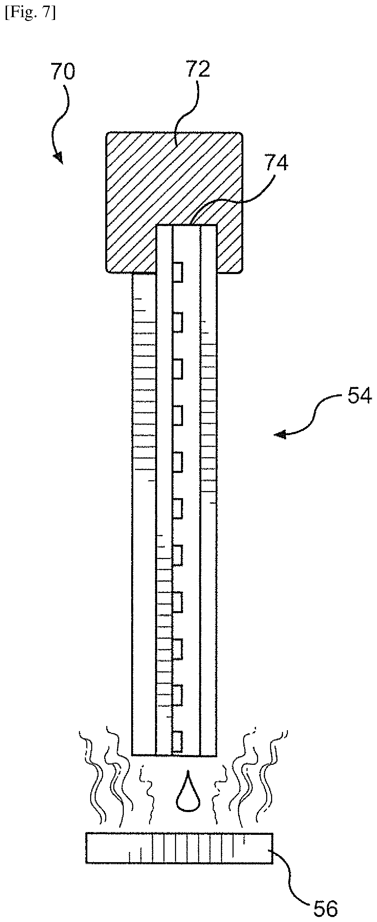

Turning now to FIG. 7, there is shown yet another embodiment of a fluid vaporization device 70. The device 70 substantially corresponds to the device 50, and includes the bubble pump 54, the vaporizer 56 and the circuit board 58. However, a fluid supply 72 is provided having an inlet 74 located at a distal end of the assembly of the bubble pump 54 and the circuit board 58 opposite the vaporizer 56.

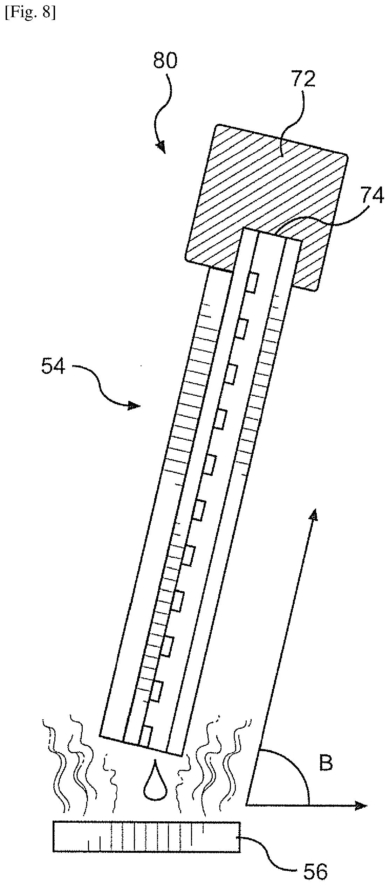

Turning now to FIG. 8, there is shown another alternate embodiment of a fluid vaporization device 80. The device 60 substantially corresponds to the device 70, and includes the fluid supply 72, bubble pump 54, and the vaporizer 56, except the circuit board 58 with the bubble pump 54 thereon is oriented at an angle B relative to the plane defined by the vaporizer 56. The angle B may vary from about 0 degrees to about 90 degrees. As in the case of the device 60, the angle B may be in one or more dimensions, as explained in connection with the angles A and A' of FIGS. 5 and 6.

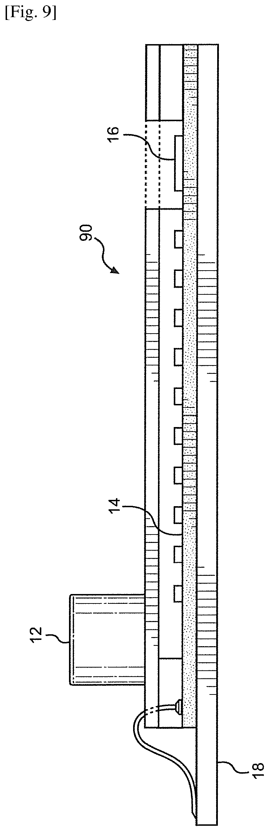

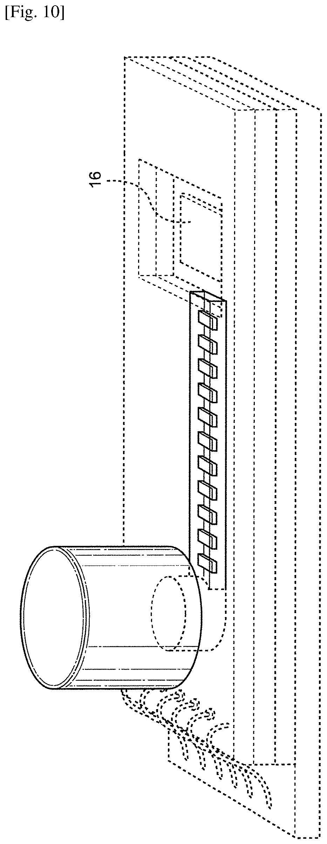

Turning now to FIGS. 9 and 10 there is shown another alternate embodiment of a fluid vaporization device 90. The device 90 substantially corresponds to the device 10, and includes the fluid supply 12, the bubble pump 14, the vaporizer 16, and the circuit board 18. However, the device 90 is constructed with the bubble pump 14 and the vaporizer 16 fabricated on the same substrate.

### (Embodiment 2)

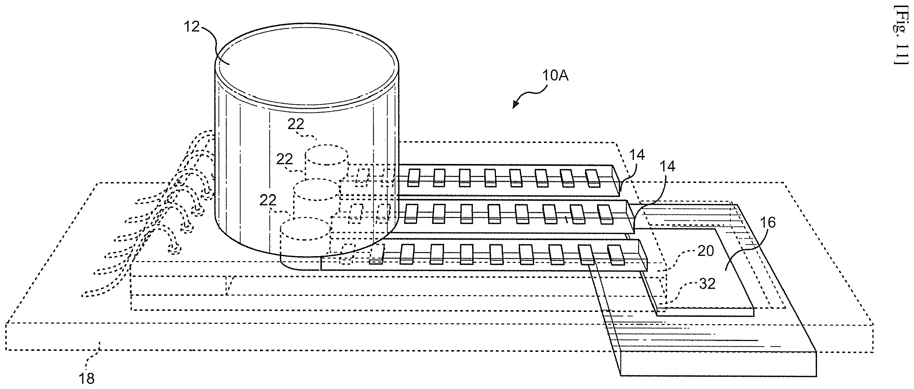

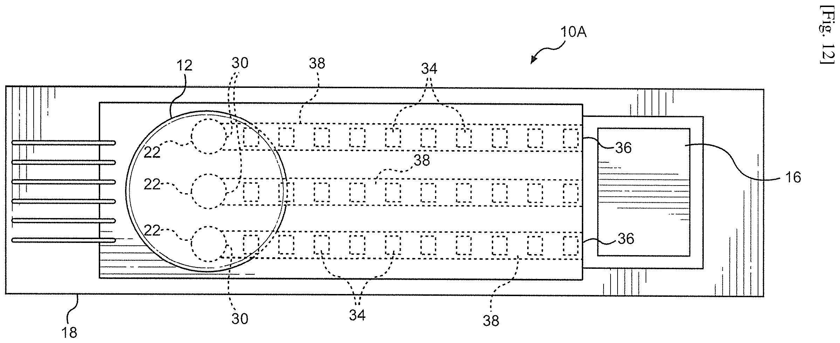

With reference to FIGS. 11-12, there is shown a fluid vaporization device 10A having a fluid supply 12, a plurality of bubble pumps 14, and a vaporizer 16. The device 10A is configured so that the bubble pumps 14 desirably transport fluid from the fluid supply 12 directly onto the vaporizer 16.

The device 10A is incorporated onto a printed circuit board 18 to provide a single assembly containing the fluid supply 12, the bubble pumps 14, and the vaporizer 16. Each of the bubble pumps 14 has a length axis that generally defines a plane, and the vaporizer 16 is provided on a substrate generally defining a plane. As will be noted, in the embodiment of FIGS. 11-12, the common plane defined by the bubble pumps 14 and the plane defined by the vaporizer 16 are substantially parallel to one another.

The fluid supply 12 is configured as a fluid storage vessel located on a cover substrate 20 of each of the bubble pumps 14. The fluid supply 12 is charged with a desired vaporizable fluid and is generally vented to the atmosphere and contains a desired volume of a fluid, typically a liquid at ambient conditions. As one example, the fluid may be a liquid of a type utilized for vapes or e-cigarettes in a volumetric amount suitable for such usage. A supply inlet 22 is defined between the fluid supply 12 and the cover substrate 20 to provide a fluidic path for desired travel of fluid from the fluid supply12 to each of the bubble pumps 14.

Each of the bubble pumps 14 is configured for pumping fluid from the fluid supply 12 to the vaporizer 16. In addition to the cover substrate 20, each bubble pump 14 includes an inlet 30, a base substrate 32, flow sequencing resistive heaters 34, and an outlet 36. During manufacture, a flow feature layer is initially deposited on the base substrate 32. The flow feature layer is then selectively etched to provide the heaters 34 and to define a flow channel 38. The base substrate 32 may be a semiconductor silicon substrate that is suitable for providing bubble pumps and logic circuits thereon. The cover substrate 20 may be made of silicon or a polymeric material such as polyimide. The resistive heaters 34 and vaporizer 16 may be made of TaAIN, TaAI or other thin film resistor material. The preferred material for the flow feature layer for providing the resistive heaters 34 is TaAIN deposited on the base substrate 32 as by sputtering. The vaporizer 16 may be formed in a similar manner.

Electrical connections and logic circuits are integrated onto the device 10A to control and operate the heaters 34 of the bubble pumps 14 and the vaporizer 16, and to otherwise control the transfer of fluid from the fluid supply 12 to the vaporizer 16. For example, voltage pulses may be applied to the heaters 34 in a desired manner to form and transport thermal bubbles of the fluid along the flow channel 38 to deliver fluid as desired to the vaporizer 16 for vaporization of the delivered fluid. Examples of preferred bubble pumps are shown in U.S. Patent No. 8,891,949, issued November 18, 2014, entitled Micro-fluidic pump, and incorporated by reference herein in its entirety.

In basic operation of the bubble pumps 14, a voltage pulse is applied to each of the heaters 34 in sequence to generate thermal bubbles in a predetermined manner. For example, every heater 34 can form a bubble from the inlet 30 to the outlet 36 of the channel 38 in sequence to transport fluid as desired from the supply 12 to the vaporization heater 16. Each heater 34 is also desirably permitted to cool down before the next firing sequence in order to prevent overheating and boiling of fluid within the bubble bump 14. The bubble pumps 14 may be operated to cooperate to provide transport of fluid to the vaporizer 16.

The vaporizer 16 is configured as a microfluidic electrical heating element designed specifically to vaporize the fluid received from the fluid supply 12. The vaporizer 16 is located adjacent and below the outlets 36 of the bubble pumps 14. A slot or other flow path is formed through the circuit board 18 for travel of fluid from the outlet 36 of the bubble pump 14 to the vaporizer 16. The vaporizer 16 has a heated fluid contact surface that is open and exposed to the air or other local environment. The heated fluid contact surface heats the received fluid to vaporize the received fluid into the atmosphere or other local environment. It will be appreciated that the vaporizer 16 may be provided by a single or multiple vaporizer structures.

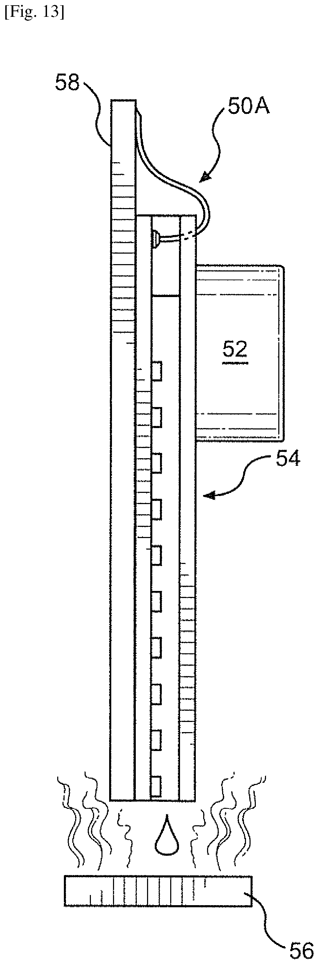

Turning now to FIG. 13, there is shown an alternate embodiment of a fluid vaporization device 50A. The device 50A has a fluid supply 52, bubble pumps 54, and a vaporizer 56. The fluid supply 52 and the bubble pumps 54 are incorporated onto a printed circuit board 58. The fluid supply 52, the bubble pumps 54, and the vaporizer 56 substantially correspond to the fluid supply 12, the bubble pumps 14, and the vaporizer 16. However, the vaporizer 56 is spaced from the end of the circuit board 58 so as to be in a plane that is substantially perpendicular to a fluid flow plane defined by the bubble pumps 54.

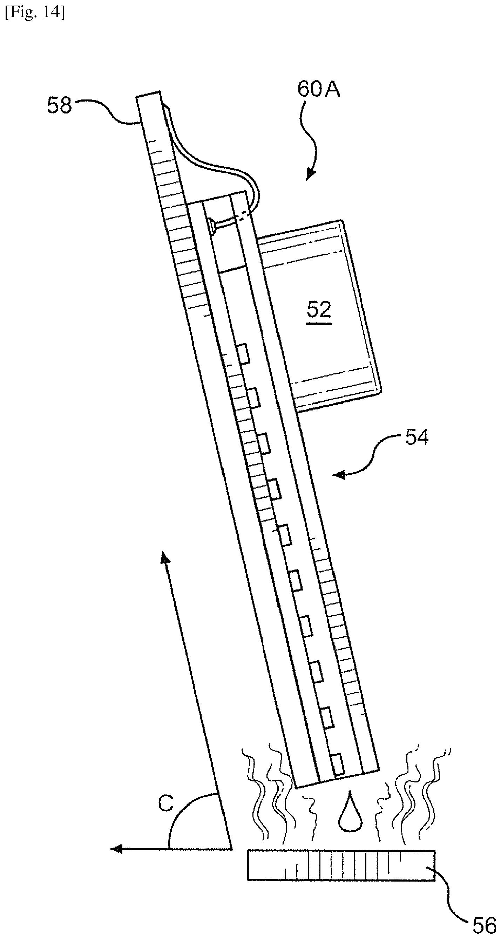

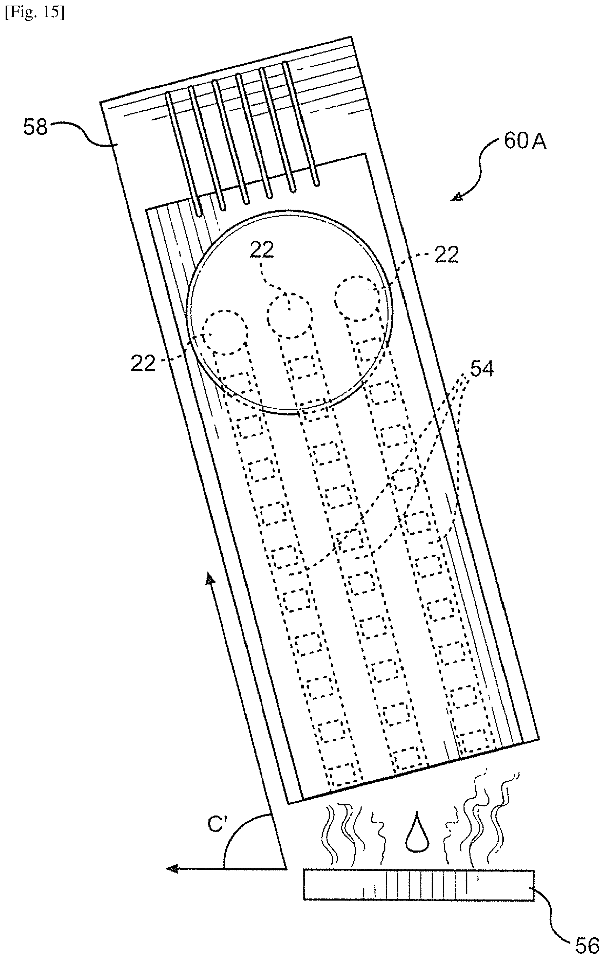

Turning now to FIGS. 14 and 15, there is shown another alternate embodiment of a fluid vaporization device 60A. The device 60A substantially corresponds to the device 50A, and includes the fluid supply 52, bubble pumps 54, and the vaporizer 56, except the circuit board 58 with the bubble pump 54 thereon is oriented at an angle C or an angle C' or both relative to a plane defined by the vaporizer 56. The angles C and C' may each vary from about 0 degrees to about 90 degrees. In this regard, it will be appreciated that the depicted angles are provided to show that the angular orientation between the bubble pumps 54 and the vaporizer 56 may be varied in any of the three dimensions.

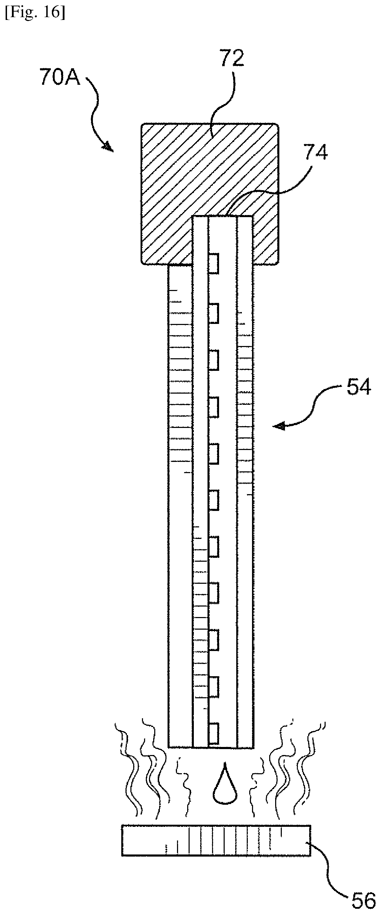

Turning now to FIG. 16, there is shown yet another embodiment of a fluid vaporization device 70A. The device 70A substantially corresponds to the device 50A, and includes the bubble pumps 54, the vaporizer 56 and the circuit board 58. However, a fluid supply 72 is provided having an inlet 74 located at a distal end of the assembly of the bubble pump 54 and the circuit board 58 opposite the vaporizer 56.

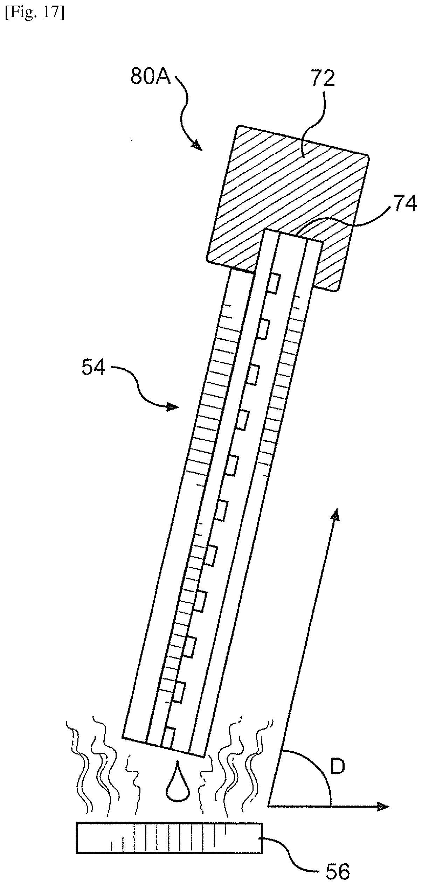

Turning now to FIG. 17, there is shown another alternate embodiment of a fluid vaporization device 80A. The device 60A substantially corresponds to the device 70A, and includes the fluid supply 72, bubble pumps 54, and the vaporizer 56, except the circuit board 58 with the bubble pumps 54 thereon is oriented at an angle D relative to the plane defined by the vaporizer 56. The angle D may vary from about 0 degrees to about 90 degrees. As in the case of the device 60A, the angle D may be in one or more dimensions, as explained in connection with the angles C and C' of FIGS. 14 and 15.

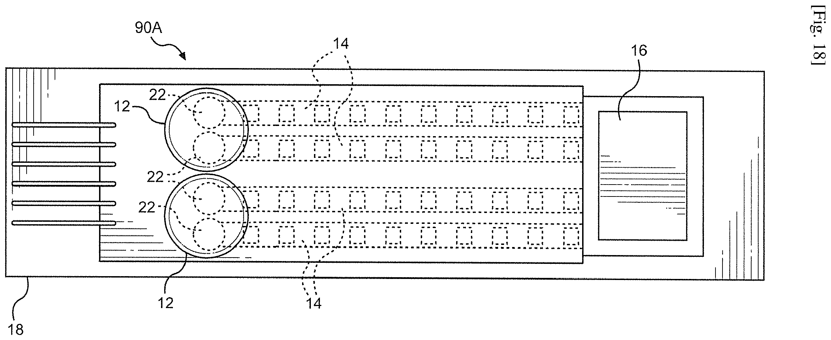

Turning now to FIG. 18, there is shown another alternate embodiment of a fluid vaporization device 90A. The device 90A substantially corresponds to the device 10A, except the device 90A includes the plurality of bubble pumps 14 in flow communication with a plurality of the fluid supplies 12. It will be appreciated that each of the fluid supplies 12 may include a different vaporizable fluid or fluids having different characteristics or mixtures of fluids.

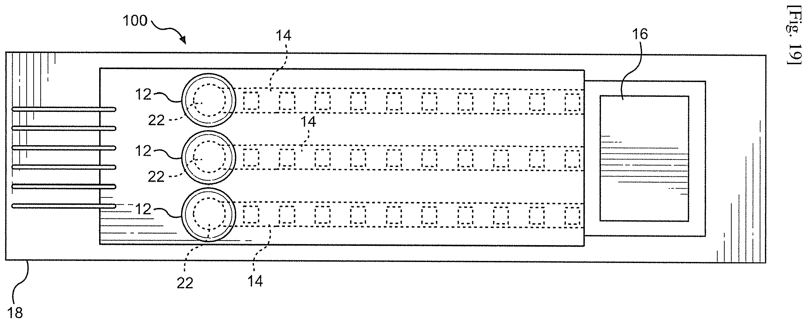

Turning now to FIG. 19, there is shown another alternate embodiment of a fluid vaporization device 100. The device 100 substantially corresponds to the device 90A, except the device 110 includes the plurality of bubble pumps 14 with the same number of fluid supplies 12. Each of the bubble pumps 14 is in flow communication with a corresponding one of the fluid supplies 12. It will be appreciated that each of the fluid supplies 12 may include a different fluid or fluids having different characteristics or mixtures of fluids.

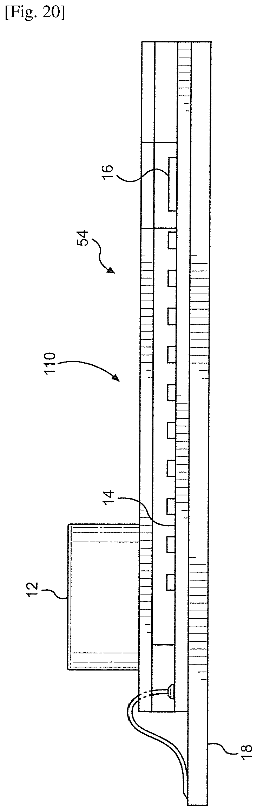

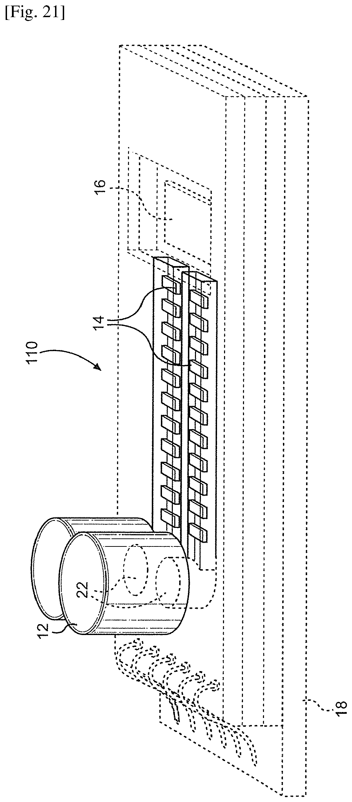

Turning now to FIGS. 20 and 21 there is shown another alternate embodiment of a fluid vaporization device 110. The device 110 substantially corresponds to the device 10A, and includes the fluid supply 12, the bubble pumps 14, the vaporizer 16, and the circuit board 18. However, the device 110 is constructed with the bubble pumps 14 and the vaporizer 16 fabricated on the same substrate.

### Reference Sign List

10, 10A, 50, 50A, 60, 60A, 70, 70A, 80, 80A, 90, 90A, 100, 110: fluid vaporization device
12, 52, 72: fluid supply
14, 54: bubble pump
16, 56: vaporizer
18, 58: printed circuit board
20: cover substrate
22: supply inlet
30, 74: inlet
32: base substrate
34: flow sequencing resistive heater
36: outlet
38: flow channel

## Claims

1. A vaporization device (10, 10A, 50, 50A, 60, 60A, 70, 70A, 80, 80A, 90, 90A, 100, 110), comprising:
a fluid supply (12, 52, 72) containing a vaporizable fluid;
a bubble pump (14, 54) operative to pump fluid from the fluid supply (12, 52, 72) to an outlet (36) of the bubble pump (14, 54); and
a fluid vaporization heater (16, 56) located adjacent the outlet (36) of the bubble pump (14, 54) to receive fluid from the bubble pump (14, 54), the fluid vaporization heater (16, 56) being operative to heat and thereby vaporize the received fluid,
wherein an angular position of the bubble pump (14, 54) relative to the fluid vaporization heater (16, 56) is variable.

2. The vaporization device (10, 10A, 50, 50A, 60, 60A, 70, 70A, 80, 80A, 90, 90A, 100, 110) of claim 1, wherein
the bubble pump (14, 54) includes:
an inlet (30, 74) in flow communication with the fluid supply (12, 52, 72) for receiving fluid therefrom;
a fluid flow path (38);
flow sequencing heaters (34) located within the fluid flow path (38); and
the outlet (36), wherein the fluid vaporization heater (16, 56) includes a heated fluid contact surface to receive fluid from the outlet (36) of the bubble pump (14, 54) and to heat and thereby vaporize the received fluid.

3. The vaporization device (10, 10A, 50, 50A, 60, 60A, 70, 70A, 80, 80A, 90A, 100) of claim 1, wherein the bubble pump (14, 54) and the fluid vaporization heater (16, 56) are fabricated on different substrates.

## Patentansprüche

1. Verdampfungsvorrichtung (10, 10A, 50, 50A, 60, 60A, 70, 70A, 80, 80A, 90, 90A, 100, 110), umfassend:
eine Fluidversorgung (12, 52, 72), die ein verdampfungsfähiges Fluid enthält;
eine Blasenpumpe (14, 54), die dafür eingerichtet ist, Fluid von der Fluidversorgung (12, 52, 72) zu einem Auslass (36) der Blasenpumpe (14, 54) zu pumpen; und
einen Fluidverdampfungserhitzer (16, 56), der neben dem Auslass (36) der Blasenpumpe (14, 54) angeordnet ist, um Fluid von der Blasenpumpe (14, 54) zu empfangen, wobei der Fluidverdampfungserhitzer (16, 56) dafür eingerichtet ist, das empfangene Fluid zu erhitzen und dadurch zu verdampfen,
wobei eine Winkelposition der Blasenpumpe (14, 54) relativ zu dem Fluidverdampfungserhitzer (16, 56) variabel ist.

2. Verdampfungsvorrichtung (10, 10A, 50, 50A, 60, 60A, 70, 70A, 80, 80A, 90, 90A, 100, 110) nach Anspruch 1, wobei die Blasenpumpe (14, 54) aufweist:
einen Einlass (30, 74) in Strömungsverbindung mit der Fluidversorgung (12, 52, 72), um Fluid von dort zu empfangen;
einen Fluidströmungspfad (38);
Strömungssequenzierungserhitzer (34), die sich innerhalb des Fluidströmungspfades (38) befinden; und
den Auslass (36),
wobei der Fluidverdampfungserhitzer (16, 56) eine beheizte Fluidkontaktfläche aufweist, um Fluid von dem Auslass (36) der Blasenpumpe (14, 54) zu empfangen und das empfangene Fluid zu erhitzen und dadurch zu verdampfen.

3. Verdampfungsvorrichtung (10, 10A, 50, 50A, 60, 60A, 70, 70A, 80, 80A, 90A, 100) nach Anspruch 1, wobei die Blasenpumpe (14, 54) und der Fluidverdampfungserhitzer (16, 56) auf unterschiedlichen Substraten hergestellt sind.

## Revendications

1. Dispositif de vaporisation (10, 10A, 50, 50A, 60, 60A, 70, 70A, 80, 80A, 90, 90A, 100, 110), comprenant :
une alimentation en fluide (12, 52, 72) contenant un fluide vaporisable ;
une pompe à bulles (14, 54) destinée à pomper le fluide provenant de l'alimentation en fluide (12, 52, 72) vers une sortie (36) de la pompe à bulles (14, 54) ; et
un réchauffeur de vaporisation de fluide (16, 56) situé à proximité de la sortie (36) de la pompe à bulles (14, 54) pour recevoir le fluide en provenance de la pompe à bulles (14, 54), le réchauffeur de vaporisation de fluide (16, 56) étant destiné à chauffer et ainsi vaporiser le fluide reçu,
dans lequel une position angulaire de la pompe à bulles (14, 54) par rapport au réchauffeur de vaporisation de fluide (16, 56) est variable.

2. Dispositif de vaporisation (10, 10A, 50, 50A, 60, 60A, 70, 70A, 80, 80A, 90, 90A, 100, 110) selon la revendication 1, dans lequel :
la pompe à bulles (14, 54) comporte :
une entrée (30, 74) en communication fluidique avec l'alimentation en fluide (12, 52, 72) pour recevoir du fluide en provenance de celle-ci ;
une voie d'écoulement de fluide (38) ;
des réchauffeurs à séquencement d'écoulement (34) situés à l'intérieur de la voie d'écoulement de fluide (38) ; et la sortie (36),
dans lequel le réchauffeur de vaporisation de fluide (16, 56) présente une surface chauffée en contact avec le fluide pour recevoir le fluide provenant de la sortie (36) de la pompe à bulles (14, 54) et pour chauffer et ainsi vaporiser le fluide reçu.

3. Dispositif de vaporisation (10, 10A, 50, 50A, 60, 60A, 70, 70A, 80, 80A, 90A, 100) selon la revendication 1, dans lequel la pompe à bulles (14, 54) et le réchauffeur de vaporisation de fluide (16, 56) sont fabriqués sur des substrats différents.
